# EUROPÄISCHE PATENTSCHRIFT

(11) **EP 3 566 049 B1**
(45) Veröffentlichungstag und Bekanntmachung des Hinweises auf die Patenterteilung: **24.02.2021**
(21) Anmeldenummer: 18730323.5
(22) Anmeldetag: 06.06.2018
(51) Int. Cl.: G01N 33/00, H05B 3/48

(54) **VORRICHTUNG ZUR FEUCHTE- UND PARTIKELABSCHEIDUNG FÜR EINEN GASSENSOR**
DEVICE FOR MOISTURE AND PARTICLE SEPARATION FOR A GAS SENSOR
DISPOSITIF DE SÉPARATION D'HUMIDITÉ ET DE PARTICULES POUR UN CAPTEUR DE GAZ

(30) Priorität: 11.07.2017 DE 102017006530
(43) Veröffentlichungstag der Anmeldung: 13.11.2019
(73) Patentinhaber: Drägerwerk AG & Co. KGaA, 23558 Lübeck (DE)
(72) Erfinder: WRUCK, Norbert, 23568 Lübeck (DE)
(86) Internationale Anmeldenummer: PCT/EP2018/064834
(87) Internationale Veröffentlichungsnummer: WO 2019/011534

(56) Entgegenhaltungen:
- WO-A1-2013/146777
- DE-A1- 2 351 815
- DE-A1-102016 101 814
- US-A1- 2002 040 599

## Beschreibung

Die vorliegende Erfindung betrifft eine Vorrichtung zur Feuchte- und Partikelabscheidung für einen Gassensor, sowie einen Gassensor mit einer solchen Vorrichtung und ein Gaswarn- oder Gaskonzentrationsmessgerät mit einem Gassensor und einer solchen Vorrichtung.

Gassensoren kommen in vielen unterschiedlichen Anwendungsgebieten zum Einsatz, darunter auch Bereiche mit teilweise extremen Umweltbedingungen. Dabei können beispielsweise hohe Staub und/oder Feuchtigkeitskonzentrationen die Standzeiten und die Messgenauigkeit von festinstallierten Gassensoren erheblich beeinflussen. Dies kann beispielsweise dann der Fall sein, wenn es zu Ablagerungen von Staub oder Feuchtigkeit im Messbereich des Sensors kommt.

Viele Gassensoren werden daher mit Schutzfiltervorrichtungen, etwa in Form von Sinterscheiben, ausgestattet. Ist der Standort des Gassensors jedoch einer hohen Staubbelastung ausgesetzt, z.B. in einem Tierstall, so ist es oft notwendig, die Schutzfiltervorrichtung regelmäßig auszutauschen.

Ebenfalls problematisch kann ein hoher Feuchtegehalt in der Umgebung sein. Sie kann genauso wie eine hohe Staubbelastung dazu führen, dass sich die Schutzfiltervorrichtung zusetzt und der Gassensor in der Folge ein zu geringes Signal ausgibt.

Vorrichtungen zur Abscheidung von Partikeln aus einem Gasgemisch sind beispielsweise aus der allgemeinen Abscheidetechnik bekannt. So offenbart DE 19934932 B4, dass mit Hilfe von Thermophorese aus dem Abgas von Verbrennungskraftmaschinen Partikel auf gekühlten Oberflächen abgeschieden werden können, und EP 2640486 A1 offenbart einen mechanischen Schutz vor groben Störpartikeln. In der Gassensorik sind ist es hingegen eher üblich, Filter zu verwenden, die bei starker Staubbeladung getauscht oder gereinigt werden müssen. Zudem ist keine der bekannten Lösungen in der Lage, gleichzeitig eine Anlagerung von Schmutzpartikeln und eine Anlagerung von Feuchtigkeit zu verhindern. Zudem gehen die meisten dieser Lösungen entweder mit einer erhöhten Trägheit des Sensors bzw. einer Minderanzeige des Sensors einher oder sie sind sehr teuer, hochempfindlich gegenüber anderweitigen Störungen oder nur mit großem Aufwand zu Installieren oder zu Warten.

US 2002/0040599 A1 beschreibt eine Schutzhülle für einen Gassensor mit einem gestreckten, festen Hüllenkörper und einer Öffnung an einer Hüllenspitze des Hüllenkörpers, wobei die Öffnung aus mindestens zwei Blendenformen besteht.

DE 10 2016 101 814 A1 beschreibt einen Gassensor mit einer röhrenförmigen Schutzeinrichtung, die an einem Metallgehäuse des Gassensors befestigt ist. Zum Gasaustausch sind Gaseinleitungsöffnung und Gasauslassöffnung an der Schutzeinrichtung vorgesehen.

DE 2 351 815 A1 beschreibt einen elektrochemischen Messfühler für die Bestimmung des Sauerstoffgehalts in Abgasen, wobei ein kegelstumpfartiger Vorsatz an einer Mantelfläche einer Schutzeinrichtung des Messfühlers beschrieben wird.

Ziel der Erfindung ist es, diese und weitere Nachteile des Standes der Technik zu überwinden und eine verbesserte Vorrichtung zur gleichzeitigen Feuchte- und Partikelabscheidung zu schaffen. Die Vorrichtung soll insbesondere einen ungehinderten Zustrom des nachzuweisenden Analyten ermöglichen und dennoch möglichst hohen Schutz vor Partikeln und Feuchtigkeitsablagerungen bieten. Darüber hinaus soll die Vorrichtung möglichst einfach und kostengünstig realisierbar sein. In einem weiteren Aspekt ist es beispielsweise Aufgabe der Erfindung einen verbesserten elektrochemischen Gassensor bereit zu stellen, wobei der Gassensor möglichst unempfindlich gegenüber Belastungen der Umgebungsatmosphäre durch Partikel und Feuchtigkeit sein soll. In noch einem weiteren Aspekt ist es beispielsweise Aufgabe der Erfindung ein Gaswarn- oder Gaskonzentrationsmessgerät bereitzustellen, wobei das Gaswarn- oder Gaskonzentrationsmessgerät möglichst unempfindlich gegenüber Belastungen der Umgebungsatmosphäre durch Partikel und Feuchtigkeit sein soll.

Zur Lösung dieser Aufgabe schlägt die Erfindung eine Vorrichtung zur Feuchte- und Partikelabscheidung entsprechend Anspruch 1, sowie einen elektrochemischen Gassensor mit einer solchen Vorrichtung entsprechend Anspruch 6 und ein Gaswarn- oder Gaskonzentrationsmessgerät mit einer solchen Vorrichtung entsprechend Anspruch 11 vor. Ausgestaltungen sind jeweils Gegenstand der abhängigen Ansprüche.

Bei einer Vorrichtung zur Feuchte- und Partikelabscheidung für einen Gassensor, schlägt die Erfindung vor, dass die Vorrichtung ein Gehäuse aufweist, in welchem ein Strömungsberuhigungsraum ausgebildet ist, wobei der Strömungsberuhigungsraum ein sensornahes Ende und ein sensorfernes Ende aufweist, wobei am sensorferne Ende eine Zutrittsöffnung ausgebildet ist und wobei am sensornahen Ende eine Austrittsöffnung ausgebildet ist, wobei der Strömungsberuhigungsraum derart beheizbar ist, dass während des Betriebes der Vorrichtung zwischen dem sensornahen Ende und dem sensorfernen Ende ein Temperaturgradient ausgebildet ist.

Ein Gassensor weist üblicherweise ein Gehäuse auf, in welchem die jeweilige Messsensorik angeordnet ist. Das Gehäuse hat typischerweise einen Einlass für das zu untersuchende Gas, im Folgenden als Gaseinlass bezeichnet. Der Gassensor kann beispielsweise ein elektrochemischer Gassensor sein. Es ist aber auch denkbar, dass eine andere Messtechnik verwendet wird. Beispielsweise kann der Gassensor auch ein katalytischer Gassensor oder ein optischer Gassensor sein.

Die Vorrichtung zur Feuchte- und Partikelabscheidung ist im einfachsten Fall eine zylinderförmige Hülse, die über den Gaseinlass des Gassensors gestülpt werden kann und diesen so gegenüber der anströmenden Umgebungsluft abschirmen kann. Die Grundfläche des Zylinders kann dabei kreisförmig sein. Sie kann aber auch einer beliebigen anderen Geometrie folgen, etwa elliptisch oder polygonal. Dabei ist es sinnvoll, wenn die Grundfläche derart gestaltet ist, dass die Geometrie der nachfolgend beschriebenen Austrittsöffnung der Geometrie der Grundfläche folgt und dass letztere derart geformt ist, dass die Austrittsöffnung möglichst passgenau über den Gaseinlass und/oder das Gehäuse des Gassensors gestülpt werden kann.

Mit anderen Worten: Die erfindungsgemäße Vorrichtung hat ein Gehäuse, das im einfachsten Fall ein Hohlzylinder ist, dessen oberes und unteres Ende offen sind und dessen Durchmesser derart bemessen ist, dass er auf den Gassensor aufgesteckt werden kann. Der Hohlzylinder hat dabei bevorzugt einen kreisförmigen Durchmesser. Denkbar ist es aber selbstverständlich auch, dass das Gehäuse eine anders geformte Grundfläche aufweist, etwa elliptisch oder polygon, wobei jedoch runde, d.h. eckenlose Grundformen bevorzugt sind.

Der Innenraum des Hohlzylinders bildet den Strömungsberuhigungsraum. Unter einem Strömungsberuhigungsraum wird im Sinne der vorliegenden Erfindung ein in dem Gehäuse der Vorrichtung ausgebildetes Volumen verstanden, durch welchen das zu analysierende Gas auf dem Weg zum Gaseinlass des Gassensors hindurchströmen muss und in welchem Verwirbelungen des anströmenden Gases soweit wie möglich abgeschwächt werden. Ist das Gehäuse ein Hohlzylinder, so bildet die Innenwand des Gehäuses Begrenzung des Strömungsberuhigungsraumes.

Der Strömungsberuhigungsraum hat ein sensornahes Ende und ein sensorfernes Ende. Das sensornahe Ende ist dasjenige Ende, das im montierten Zustand dem Gaseinlass des Gassensors zugewandt ist. Das sensorferne Ende ist das Ende, das im montierten Zustand vom Gaseinlass des Gassensors entfernt angeordnet ist. Sowohl das sensorferne Ende als auch das sensornahe Ende weisen eine Öffnung auf. Der Durchmesser der jeweiligen Öffnung kann dabei entweder dem inneren Durchmesser des Hohlzylinders entsprechen oder kleiner als der innere Durchmesser des Hohlzylinders sein. Bevorzugt ist jedoch, wenn der Durchmesser der Öffnung am sensornahen Ende derart ausgebildet ist, dass die Öffnung den Gassensor aufnehmen kann. Weiterhin bevorzugt ist es, wenn der Durchmesser der Öffnung am sensorfernen Ende möglichst groß gestaltet ist. Dies hat den Vorteil, dass das zu untersuchende Gas weitestgehend ungehindert in den Strömungsberuhigungsraum eintreten kann. Die Öffnung am sensorfernen Ende ist mithin eine Zutrittsöffnung, durch welche das zu untersuchende Gas in den Strömungsberuhigungsraum eintreten kann. Die Öffnung am sensornahen Ende ist eine Austrittsöffnung, durch welche das zu untersuchende Gas aus dem Strömungsberuhigungsraum austreten und in den Gaseinlass des Gassensors eintreten kann.

Erfindungsgemäß ist der Strömungsberuhigungsraum beheizbar. Dies ist realisiert, indem das Gehäuse der Vorrichtung eine Heizung aufweist, so dass die Wandung, die den Strömungsberuhigungsraum begrenzt, erwärmt werden kann. Dabei bildet sich erfindungsgemäß ein Temperaturgradient aus. Mit Hilfe des Temperaturgradienten kann erreicht werden, dass Feuchtigkeitströpfchen, die aus der eintretenden Gasphase kondensieren könnten, vom sensornahen Ende des Strömungsberuhigungsraum ferngehalten werden.

Man erkennt mithin, dass die erfindungsgemäße Vorrichtung sowohl membranfrei als auch filterfrei funktioniert. Gleichzeitig bietet die Vorrichtung jedoch einen verbesserten Schutz des Gaseinlasses des Gassensors vor Partikel- und Feuchtigkeitsanlagerung. Dabei schützt der Strömungsberuhigungsraum mit der sensorfern angeordneten Zutrittsöffnung vor der Anlagerung von Schmutzpartikeln und anströmenden Feuchtigkeitströpfchen. Gleichzeitig kann durch das Beheizen des Strömungsberuhigungsraumes, insbesondere der Wandung des Strömungsberuhigungsraumes, effektiv verhindert werden, dass aus der Gasphase kondensierende Feuchtigkeit sich am Gaseinlass des Gassensors anlagern kann.

Letzteres wird insbesondere dadurch unterstützt, dass die Innenwand des Strömungsberuhigungsraumes eine kondensationsverhindernde Oberfläche aufweist. Die Innenwand des Strömungsberuhigungsraumes entspricht dabei der Innenwand des Gehäuses. Unter einer kondensationsverhindernden Oberfläche wird eine Oberfläche verstanden, welche der Kondensation von Feuchtigkeitspartikeln aus der Gasphase durch Wärmezufuhr entgegenwirkt. Beispielsweise kann die Innenwand des Strömungsberuhigungsraumes mit Metall beschichtet sein oder aus Metall bestehen. So kann das Gehäuse beispielsweise eine Edelstahlhülse sein. Denkbar ist auch, dass die Innenwand mit einer bevorzugt korrosionsfesten Epoxydharzschicht beschichtet ist. Ist eine solche Beschichtung vorhanden, so versteht es sich von selbst, dass Beschichtungsmaterialien bevorzugt sind, die keine adsorbierende Wirkung gegenüber dem jeweils nachzuweisenden Analyten haben. Man erkennt, dass auf diese Weise die Kondensation von unerwünschter Feuchtigkeit im Bereich des sensornahen Endes vermieden wird und dass so verhindert werden kann, dass sich Flüssigkeit im Bereich des Gaseinlasses des Gassensors absetzt.

Zum Beheizen des Strömungsberuhigungsraumes ist erfindungsgemäß in der Wand des Gehäuses wenigstens ein Heizelement angeordnet. Die Wand des Gehäuses bildet dabei wenigstens teilweise die Wand des Strömungsberuhigungsraumes. Mit anderen Worten, in der Wand des Strömungsberuhigungsraumes ist wenigstens ein Heizelement angeordnet. Das Heizelement kann bspw. eine Heizwendel, eine Folienheizung eine Dickschichtheizung oder eine Dünnschichtheizung sein. Ein solches Heizelement kann platzsparend entlang des Umfanges der Wand des Gehäuses oder innerhalb der Gehäusewandung angeordnet werden. Auf diese Weise kann entlang der Wand des Gehäuses und damit entlang der Innenwand des Strömungsberuhigungsraumes ein Temperaturgradient erzeugen. Zudem kann die Vorrichtung relativ platzsparend ausgebildet sein.

Vorteilhaft ist es auch, wenn die Heizleistung des Heizelementes ungleichmäßig über den Umfang des Gehäuses verteilt ist. Dazu kann das Heizelement einseitig entlang des Umfanges angeordnet sein. Alternativ kann das Heizelement auch unsymmetrisch angesteuert werden. Eine ungleichmäßige Verteilung der Heizleistung entlang des Umfanges bietet den Vorteil, dass innerhalb des Strömungsberuhigungsraumes eine natürliche Konvektion induziert wird, durch welche nur warmes, Tröpfchen freies Gas an den Gassensor geführt wird.

Erfindungsgemäß weist die Vorrichtung einen Prallschutz auf. Dieser dient als mechanische Barriere für heranströmende Partikel. Der Prallschutz kann dabei die Zutrittsöffnung der Vorrichtung begrenzen. Die Zutrittsöffnung wird mithin durch die Gehäuseöffnung und durch den vorgelagerten Prallschutz begrenzt. Die Größe der Zutrittsöffnung wird mithin von der Größe der Gehäuseöffnung maßgeblich mitbestimmt. Der Prallschutz ist vorteilhafterweise in einem Abstand von der Öffnung des Gehäuses angeordnet, so dass zwischen dem Prallschutz und der Öffnung des Gehäuses, die gleichzeitig die Öffnung des Strömungsberuhigungsraumes ist, ein Spalt ausgebildet ist. Dieser Spalt bildet dann die Zuströmöffnung. Man erkennt, dass mit Hilfe des Prallschutzes der Gaseinlass des Gassensors effektiv vor Partikeln und Feuchtigkeitstropfen geschützt werden, die durch konvektiven Transport von unten in die Vorrichtung einströmen und unmittelbar auf den Gaseinlass prallen würden. Dabei weist der Prallschutz erfindungsgemäß einen Prallschutzteller auf. Dieser ist bevorzugt in einem Abstand vom sensorfernen Ende angeordnet, wobei die Zutrittsöffnung zwischen dem Prallschutzteller und dem sensorfernen Ende ausgebildet ist. Der Prallschutzteller kann beispielsweise eine Platte sein, die vor der sensorfernen Öffnung des Strömungsberuhigungsraumes, mit anderen Worten am sensorfernen Ende des Gehäuses, angeordnet ist. Denkbar ist, dass der Prallschutzteller eine konkave oder konvexe Oberfläche aufweist. Die konkave Oberfläche kann auf der von der Öffnung des Strömungsberuhigungsraumes abgewandten Seite ausgebildet sein. Die konvexe Oberfläche kann auf der der sensorfernen Öffnung des Strömungsberuhigungsraumes zugewandten Seite ausgebildet sein. Anstelle der konkaven Oberfläche auf der von der Öffnung abgewandten Seite, kann der Prallschutzteller auf dieser Seite auch planar ausgebildet sein. Die konkave oder konvexe Gestaltung der Oberfläche des Prallschutztellers kann dazu beitragen, dass das anströmende Gasgemisch optimal in den Strömungsberuhigungsraum eingeleitet wird, wobei gleichzeitig anströmende Staubpartikel, die von dem Prallschutzteller abprallen in eine Richtung, die möglichst vom Strömungsberuhigungsraum weg weist, reflektiert werden können.

In einer weiteren Ausführungsvariante ist vorgesehen, dass der Prallschutz wenigstens ein ringförmiges Element umfasst, das zwischen dem Prallschutzteller und dem sensorfernen Ende angeordnet ist. Beispielsweise kann das ringförmige Element eine stumpfkegelige äußere Form aufweisen mit einem erste Ende, das einen größeren Umfang aufweist und einem zweiten Ende, das einen kleineren Umfang aufweist. In anderen Worten, das ringförmige Element kann trichterförmig ausgebildet sein. Dabei ist es zweckmäßig, das ringförmige Element derart zwischen der Öffnung des Gehäuses und dem Prallschutzteller anzuordnen, dass der größere Umfang zur Öffnung des Gehäuses und der kleinere Umfang dem Prallschutzteller zugewandt ist. Seitlich anströmende Partikel können auf diese Weise auf die schräge Außenfläche aufprallen und werden von dort von der Öffnung des Gehäuses weg abgelenkt. Dieses ringförmige Element bietet einen zusätzlichen Schutz bei lateraler Anströmung des zu untersuchenden Gasgemischs. Man erkennt in jedem Fall, dass es vorteilhaft ist, wenn der Prallschutz sowohl einen Prallschutzteller als auch wenigstens ein ringförmiges Element aufweist.

Denkbar ist auch, dass der Prallschutz mehrere ringförmige Elemente umfasst, die zwischen dem Prallschutzteller und dem sensorfernen Ende angeordnet sind. Auf diese Weise wird der Schutz gegenüber lateraler Anströmung besonders effektiv gestaltet. Wobei das ringförmige Element auch durch Vertikallamellen ersetzt sein kann. Mit anderen Worten an Stelle des ringförmigen Elementes ist auch ein lamellenförmiges Element denkbar. Die Lamellen können dabei parallel zur Richtung, die vom sensorfernen Ende zum sensornahen Ende verläuft, ausgerichtet sein. Denkbar ist auch, dass sie leicht geneigt gegenüber dieser Richtung verlaufen, so dass sie - wie zuvor für das ringförmige Element beschrieben - eine trichterförmige Struktur bilden. Insbesondere die Kombination der verschiedenen Prallschutzelemente, also von einem oder mehreren ring- oder lamellenförmigen Elementen mit einem Prallschutzteller, kann sicher stellen, dass sowohl bei Anströmung von unten, von der Seite sowie von schräg oben oder schräg unten ein Schutz vor eindringenden Partikeln gewährleistet ist, gleichzeitig aber der Analyt in den Strömungsberuhigungsraum eindringen kann.

In einem weiteren Aspekt schlägt die Erfindung einen Gassensor mit einer Vorrichtung zur Feuchte- und Partikelabscheidung, wie sie oben beschrieben wurde, vor. Ein Gassensor, der mit einer solchen Vorrichtung ausgerüstet ist, zeichnet sich durch eine längere Standzeit und ein zuverlässigeres Messsignal auch in stark staubbelasteten oder hochfeuchten Umgebungen aus. Der Gassensor muss weniger häufig gewartet werden, insbesondere setzt sich die Sinterscheibe nicht so häufig zu und die dahinterliegende Messsensorik ist besser geschützt. Als besonders günstig hat sich die erfindungsgemäße Kombination von Gassensor und Vorrichtung zur Feuchte- und Partikelabscheidung bei der Verwendung von elektrochemischen Gassensoren erwiesen. Es ist jedoch ohne Weiteres auch denkbar, dass der Gassensor ein katalytischer Gassensor oder ein optischer Gassensor oder ein Gassensor entsprechend noch einem anderen Messprinzip ist.

Es sind verschiedene Varianten vorstellbar, mit denen die Montage der Vorrichtung zur Feuchte- und Partikelabscheidung am Gassensor erfolgen kann. So ist in einer ersten Variante denkbar, dass das Gehäuse der Vorrichtung zur Feuchte- und Partikelabscheidung stoffschlüssig mit dem Gehäuse des Gassensors verbunden ist. Denkbar ist auch, dass das Gehäuse der Vorrichtung zur Feuchte- und Partielabscheidung kraft- oder formschlüssig mit dem Gehäuse des Gassensors verbunden ist.

Unter einer stoffschlüssigen Verbindung wird beispielsweise das Verbinden durch kleben, schweißen oder der gleichen verstanden. Auch eine einstückige Ausführung mit dem Gehäuse des Gassensors ist denkbar. Beispielsweise kann die Vorrichtung auch ein Teil des Gassensors oder des Gaswarngerätes sein. Unter einer kraft- oder formschlüssigen Verbindung wird ein reibschlüssiges Aufstecken, Aufschrauben oder ähnliches verstanden. Eine solche Verbindung hat beispielsweise den Vorteil, dass die Vorrichtung einfach ausgetauscht und gewartet werden kann.

In einer besonders günstigen Ausführungsvariante ist es vorgesehen, dass zwischen dem Gehäuse des Gassensors und dem Gehäuse der Vorrichtung Abströmschlitze ausgebildet sind. Mit Hilfe dieser Abströmschlitze können die Strömungsverhältnisse innerhalb des Strömungsberuhigungsraumes zusätzlich verbessert werden, so dass die Ansprechzeit des Gassensors deutlich verkürzt werden kann. Wird die Vorrichtung auf das Gehäuse des Gassensors aufgesteckt, so können die Abströmschlitze beispielsweise dadurch gebildet werden, dass die Austrittsöffnung der Vorrichtung, welche den Gassensor beim Aufstecken aufnimmt, geringfügig größer ist als die der äußere Umfang des Teils des Gassensors, der in die Austrittsöffnung gesteckt wird. Die Austrittsöffnung weist dann beispielsweise Haltestege auf, die ihren Durchmesser an einigen wenigen Punkten verkleinert und die passgenau mit dem Gehäuse des Gassensors in Reibschluss gebracht werden können. Auf diese Weise wird die Vorrichtung mit Hilfe der Haltestege am Gassensor reibschlüssig festgehalten.

In jedem Fall ist es günstig, wenn das Volumen des Strömungsberuhigungsraumes derart bemessen ist, dass die Ausbildung eines Konzentrationsgradienten des Analyten zwischen der Zutrittsöffnung und dem sensornahen Ende verhindert wird. In diesem Zusammenhang wird unter der Verhinderung der Bildung eines Konzentrationsgradienten des Analyten verstanden, dass verhindert wird, dass sich ein messtechnisch bedeutsamer Konzentrationsgradient ausbildet. Ein messtechnisch bedeutsamer Konzentrationsgradient ist in diesem Zusammenhang ein Konzentrationsgradient, der dazu führt, dass der Gassensor einen niedrigeren Messwert anzeigt, als tatsächlich in der der Umgebungsatmosphäre vorhanden ist. In seltenen Fällen ist auch denkbar, dass der Gassensor einen zu hohen Messwert anzeigt. In beiden Fällen würde ein Konzentrationsgradient, der eine solche Messwertabweichung zur Folge hat als messtechnisch bedeutsamer Konzentrationsgradient angesehen. Liegt hingegen ein derart minimaler Konzentrationsgradient vor, dass der Messwert den der Gassensor liefert nicht beeinflusst wird, so wird der Konzentrationsgradient als messtechnisch nicht bedeutend angesehen. In jedem Fall ist denkbar, dass das Volumen des Strömungsberuhigungsraumes um ein Vielfaches größer ist als das eigentliche Zutrittsvolumen vor dem Gassensor. Ist der Gassensor ein elektrochemischer Gassensor, so ist es beispielsweise vorstellbar, dass das Volumen des Strömungsberuhigungsraumes wenigstens 2 cm³ und höchstens 200 cm³ beträgt, bevorzugt wenigstens 5 cm³ oder mehr und höchstens 100 cm³ oder weniger, besonders bevorzugt wenigstens 10 cm³ oder mehr und höchstens 75 cm³ oder weniger, ganz besonders bevorzugt wenigstens 20 cm³ oder mehr und höchstens 50 cm³ oder weniger beträgt. In jedem Fall kann so verhindert werden, dass eine unzulässige Behinderung des physikalischen Messvorgangsentsteht. Die Vorrichtung reinigt zwar die Umgebungsatmosphäre von Partikeln und kondensierender Feuchte, verringert aber nicht die Menge des anströmenden Analyten.

In noch einem weiteren Aspekt schlägt die Erfindung ein Gaswarn- oder Gaskonzentrationsmessgerät mit einem Gassensor und einer Vorrichtung zur Feuchte- und Partikelabscheidung wie jeweils zuvor beschrieben vor. Unter einem Gaswarn- oder Gaskonzentrationsmessgerät wird in diesem Zusammenhang eine Vorrichtung verstanden, die wenigstens einen Gassensor aufweist und die entweder ein Gaswarngerät oder ein Gaskonzentrationsmessgerät oder eine Kombination aus beidem sein kann. Ein Gaswarngerät ist dabei eine Vorrichtung, die bei Vorhandensein eines bestimmten Gases oder mehrerer verschiedener Gase in einem Gasgemisch, etwa der Umgebungsatmosphäre der Vorrichtung, einen Alarm ausgibt. Ein Gaskonzentrationsmessgerät ist in diesem Zusammenhang eine Vorrichtung, die die Konzentration eines bestimmten Gases oder mehrerer verschiedener Gase in einem Gasgemisch feststellt. Ein Gaskonzentrationsmessgerät kann dabei auch ein Gaswarngerät sein. Dabei ist denkbar, dass die Vorrichtung zur Feuchte- und Partikelabscheidung entweder am Gehäuse des Gassensors oder an einem dafür vorgesehenen Gehäuseabschnitt des Gaswarn- oder Gaskonzentrationsmessgerätes montiert ist. Beispielsweise ist denkbar, dass der Gassensor in einer Aufnahme des Gaswarn- oder Gaskonzentrationsmessgerätes angeordnet ist und die Vorrichtung stoff-, kraft- oder formschlüssig mit der Aufnahme verbunden ist, so dass das sensornahe Ende der Vorrichtung dem Gaseinlass des Gassensors zugewandt ist und/oder diesen umfasst. Dabei können sowohl zwischen der Vorrichtung zur Feuchte- und Partikelabscheidung und dem Gassensor als auch zwischen der Vorrichtung zur Feuchte- und Partikelabscheidung und dem Gaswarn- oder Gaskonzentrationsmessgerät die zuvor beschriebenenen Abströmschlitze ausgebildet sein.

Weitere Merkmale, Einzelheiten und Vorteile der Erfindung ergeben sich aus dem Wortlaut der Ansprüche, sowie aus der folgenden Beschreibung von Ausführungsbeispielen anhand der Zeichnungen. Es zeigen:
- Fig. 1a: eine schematische Seitenansicht einer Vorrichtung zur Feuchte- und Partikelabscheidung
- Fig. 1b: einen Längsschnitt entlang der Linie A-A durch die Vorrichtung entsprechend Fig. 1a
- Fig. 1c: einen Querschnitt entlang der Linie B-B durch die Vorrichtung entsprechend den Figuren 1a und 1b
- Fig. 2a: eine schematische Seitenansicht einer Vorrichtung zur Feuchte- und Partikelabscheidung
- Fig. 2b: einen Längsschnitt entlang der Linie A'-A' durch die Vorrichtung entsprechend Fig. 2a
- Fig. 3: eine schematische Seitenansicht einer Vorrichtung zur Feuchte- und Partikelabscheidung
- Fig. 4: einen Längsschnitt durch einen Gassensor mit einer Vorrichtung zur Feuchte- und Partikelabscheidung
- Fig. 5a: einen Längsschnitt durch einen Gassensor mit einer Vorrichtung zur Feuchte- und Partikelabscheidung
- Fig. 5b: einen Querschnitt durch den Gassensor und die Vorrichtung zur Feuchte- und Partikelabscheidung entsprechend Fig. 5a entlang der Linie C-C
- Fig. 6: eine schematische Ansicht eines Gaswarn- oder Gaskonzentrationsmessgerätes mit einem Gassensor und einer Vorrichtung zur Feuchte- und Partikelabscheidung
- Fig. 7: verschiedene Messkurven bei Ausgestaltung der Vorrichtung mit und ohne Abluftschlitzen im Vergleich

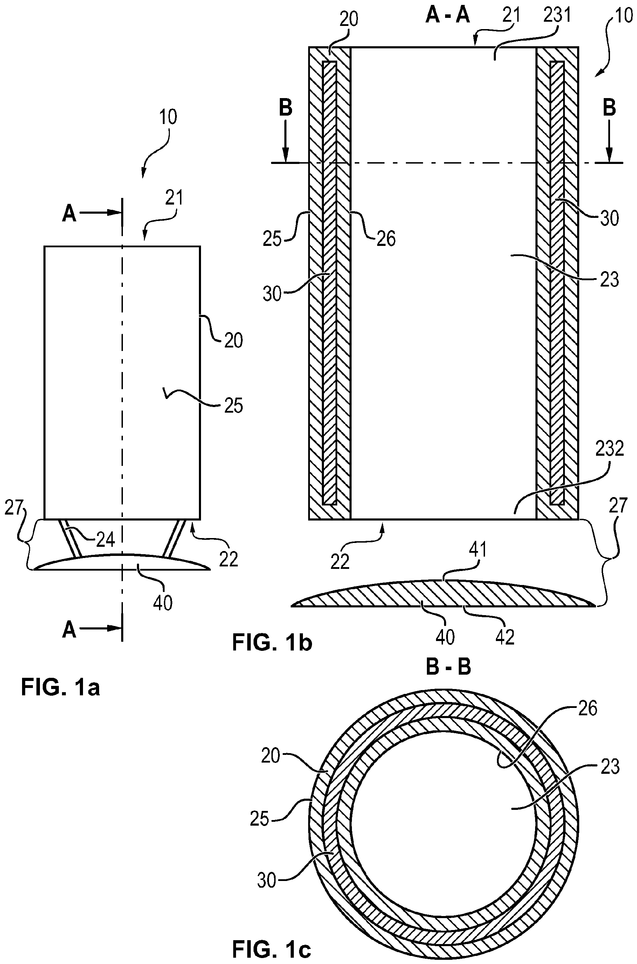

Fig. 1a zeigt eine Vorrichtung zur Feuchte- und Partikelabscheidung 10 (im Folgenden kurz als die Vorrichtung 10 bezeichnet). Die Vorrichtung 10 hat ein Gehäuse 20. An dem Gehäuse 20 ist mit Hilfe von Haltestegen 24 ein Prallschutzteller 40 angebracht. Das Gehäuse 20 hat eine Außenwand 25 und eine (in Fig. 1a nicht dargestellte) Innenwand 26. Das Gehäuse 20 weist weiterhin eine Austrittsöffnung 21 auf und eine Öffnung 22, die gegenüber dem Prallschutzteller 40 ausgebildet ist. Zwischen dem Gehäuse 20 und dem Prallschutzteller 40 ist eine Zutrittsöffnung 27 ausgebildet. Durch die Zutrittsöffnung 27 kann die Umgebungsatmosphäre zur Öffnung 22 strömen und so in das Innere des Gehäuses 20 gelangen.

In Fig. 1b erkennt man, dass das Gehäuse 20 die Form eines Hohlzylinders hat. Das Innere des Gehäuses wird von einem als Strömungsberuhigungsraum 23 bezeichneten Volumen gebildet. Der Strömungsberuhigungsraum 23 wird durch die Innenwand 26 des Gehäuses 20 begrenzt. Im montierten Zustand der Vorrichtung 10 bildet die Austrittsöffnung 21 das sensornahe Ende 231 des Strömungsberuhigungsraumes 23, während die Öffnung 22 das sensorferne Ende 232 des Strömungsberuhigungsraumes 23 bildet. In der Wand des Gehäuses 20 ist ein Heizelement 30 angeordnet. In Fig. 1c erkennt man, dass das Heizelement 30 entlang des Umfanges des Gehäuses 20 angeordnet ist. Das Heizelement 30 ist in allen gezeigten Abbildungen nur schematisch dargestellt. Es kann sowohl aus einer Folienheizung, als auch aus einer Heizwendel, stabförmigen Heizelementen, einer Dickschichtheizung oder einer Dünnschichtheizung bestehen. Mit Hilfe des Heizelementes 30 ist der Strömungsberuhigungsraum 23 beheizbar. Dabei ist ein Temperaturgradient erzeugbar, der sich vom sensornahen Ende 231 zum sensorfernen Ende 232 erstreckt.

Im Betrieb der Vorrichtung 10 strömt die zu untersuchende Umgebungsatmosphäre durch die Zutrittsöffnung 27 und die Öffnung 22 in den Strömungsberuhigungsraum 23. Dabei prallen Staubpartikel, die normalerweise durch konvektiven Transport von unten in den Strömungsberuhigungsraum 23 hineintransportiert würden, am Prallschutzteller 40 ab. Die Vorrichtung 10 verhindert mithin, dass Staubpartikel in den Bereich des sensornahen Endes 231 transportiert werden. Auf gleiche Weise schützt der Prallschutzteller 40 auch vor dem Eindringen von Feuchtigkeitströpfchen, die über konvektiven Transport in den Bereich der Vorrichtung 10 gelangen.

Mit Hilfe des Heizelementes 30 wird zudem erreicht, dass Feuchtigkeit, die im Bereich des Strömungsberuhigungsraumes 23 als Tröpfchen vorliegt oder aus der Gasphase auskondensiert, sich nicht im Bereich des sensornahen Endes 231 ablagert. Durch den Temperaturgradienten, der mit Hilfe des Heizelementes 30 erzeugt wird, wird erreicht, dass die Kondensation von Feuchtigkeit aus der Gasphase durch Anhebung der Temperatur vermieden wird. Eventuell bereits anströmende Mikrotropfen verdunsten in diesem Bereich und könne sich nicht am Gassensor anlagern.

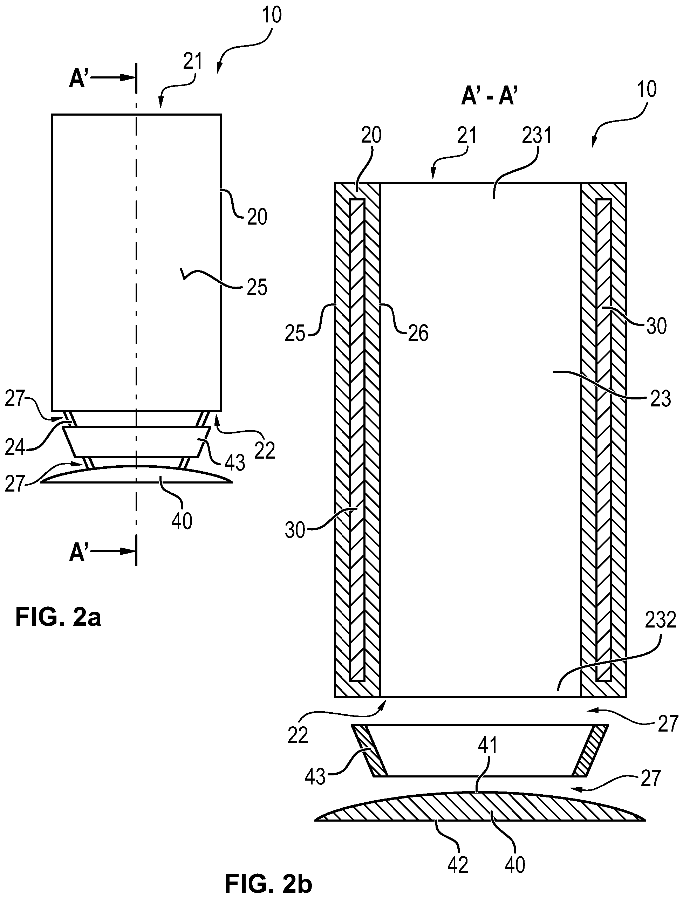

Das in den Figuren 2a und 2b gezeigte Ausführungsbeispiel entspricht dem bereits oben in Bezug auf Fig. 1 a , 1 b und 1c beschriebenen Ausführungsbeispiel. Gleiche Bezugszeichen bezeichnen insofern auch gleiche Bauteile. Das Ausführungsbeispiel entsprechend den Figuren 2a und 2b weist jedoch zusätzlich noch ein ringförmiges Element 43 auf. Dieses ringförmige Element 43 dient ebenfalls als Prallschutzelement. Es unterstützt mithin die Funktion des Prallschutztellers 40. Man erkennt, dass das ringförmige Element 43 trichterförmig ausgebildet ist. Das ringförmige Element 43 begrenzt gemeinsam mit dem Prallschutzteller 40 die Zutrittsöffnung 27.

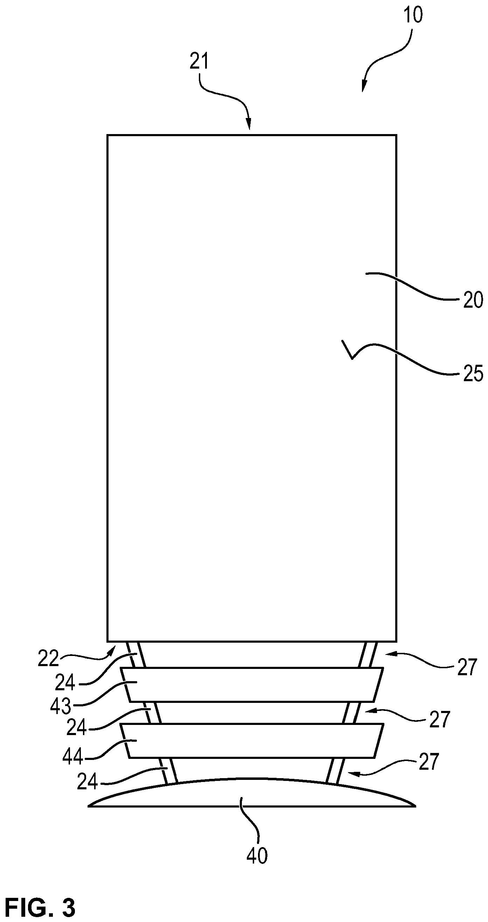

Auch das in Figur 3 dargestellte Ausführungsbeispiel entspricht dem bereits oben in Bezug auf die Fig. 1a, 1b und 1c sowie 2a und 2b beschriebenen Ausführungsbeispiel und gleiche Bezugszeichen bezeichnen insofern auch hier gleiche Bauteile. Das Ausführungsbeispiel entsprechend Fig. 3 weist gegenüber den zuvor beschriebenen Ausführungsbeispielen noch ein weiteres ringförmiges Element 44 auf. Der Prallschutz wird mithin in diesem Ausführungsbeispiel von dem Prallschutzteller 40, dem ersten ringförmigen Element 43 und dem zweiten ringförmigen Element 44 gebildet. Alle diese Elemente sind mit Hilfe von Haltestegen 24 am Gehäuse 20 der Vorrichtung 10 befestigt. Die Zutrittsöffnung 27 wird durch alle diese Elemente begrenzt und geformt.

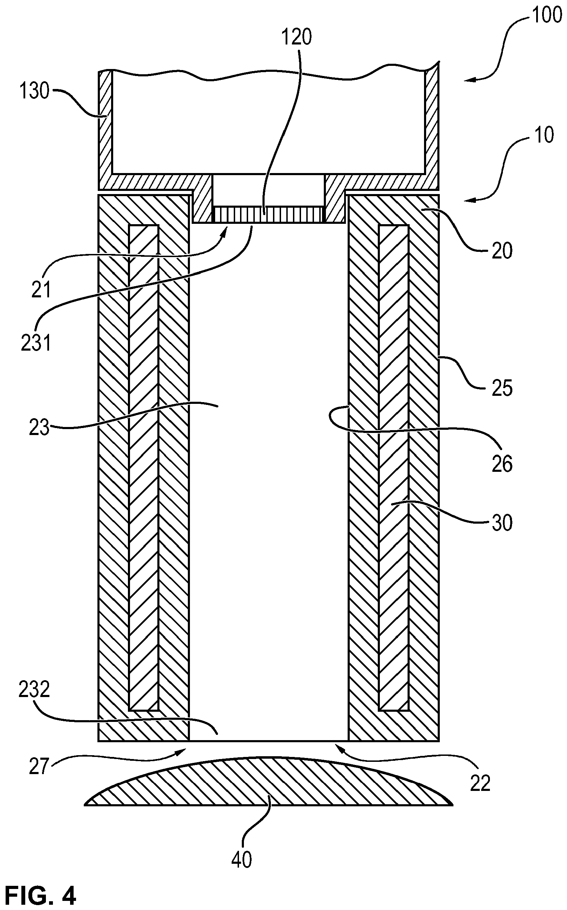

In Fig. 4 erkennt man, wie die erfindungsgemäße Vorrichtung 10 an einem Gassensor 100 montiert ist. Im dargestellten Beispiel entspricht die Vorrichtung 10 dem in den Figuren 1a, 1b und 1c gezeigten Ausführungsbeispiel. Es versteht sich jedoch von selbst, dass dies nur exemplarisch ist und dass anstelle dieser Variante der Vorrichtung 10 auch die in den Figuren 2a, 2b, 2c oder 3 dargestellte Variante am Gassensor 100 montiert sein kann. In jedem Fall erkennt man, dass der Gassensor 100 ein Gehäuse 130 aufweist. Die Vorrichtung 10 ist derart gestaltet, dass die Austrittsöffnung 21 wenigstens einen Teil des Gehäuses 130 formschlüssig umgreift. Man erkennt, dass die Vorrichtung 10 auf diese Weise auf den Gassensor 100 aufgesteckt ist. Im Gassensor 100 ist ein Zuströmbereich 110 ausgebildet, der von einer Schutzfiltervorrichtung 120 vor dem Eindringen von Staub oder Feuchtigkeit geschützt wird. Die Vorrichtung 10 schützt wiederum die Schutzfiltervorrichtung 120 vor anströmendem Staub oder Feuchtigkeit und verhindert erfindungsgemäß, dass sich die Schutzfiltervorrichtung 120 mit Staub oder Feuchtigkeit zusetzt.

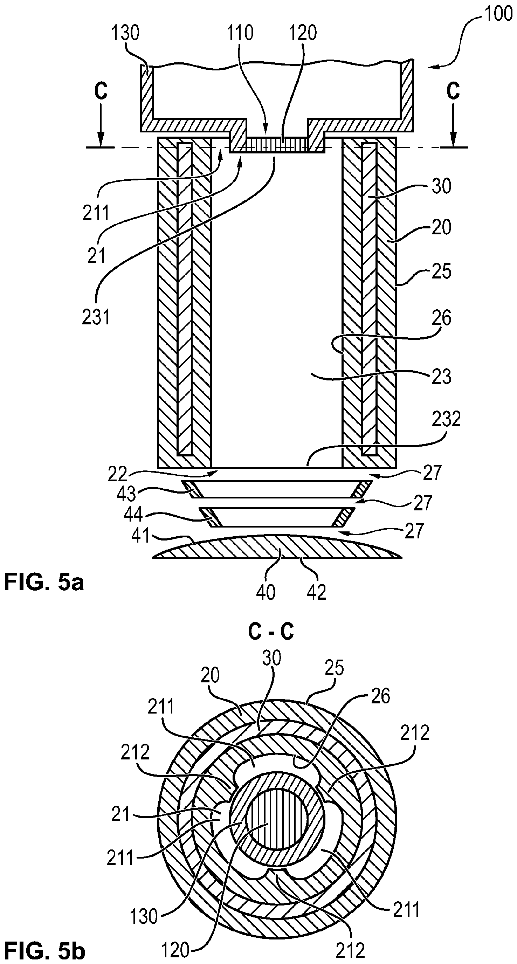

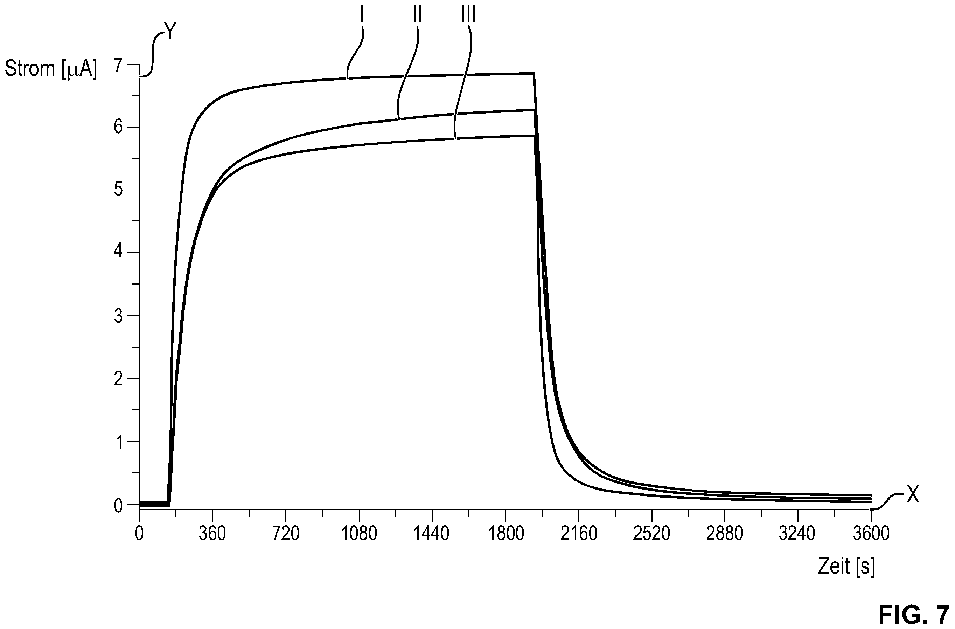

Die Figuren 5a und 5b zeigen eine Ausführungsvariante, bei welcher zwischen dem Gassensor 100 und der Vorrichtung 10 im montierten Zustand Abströmschlitze 211 ausgebildet sind. Im Querschnitt entlang der Linie C-C erkennt man, dass der Durchmesser der Austrittsöffnung 21 größer ist als der Umfang des Gehäuses 130 des Gassensors 100 in dem Bereich, der von der Austrittsöffnung 21 umfasst wird. An der Innenwand 26 des Gehäuses 20 der Vorrichtung 10 sind jedoch Haltestege 212 ausgebildet, die das Gehäuse 130 des Gassensors 100 umfassen und mit diesem in Kontakt stehen. Auf diese Weise kann die Vorrichtung 10 auf den Gassensor 100 aufgesteckt werden. Zwischen dem Gehäuse 20 der Vorrichtung 10 und dem Gehäuse 130 des Gassensors 100 bilden sich dabei die Abströmschlitze 211. Man erkennt in Figur 7, dass diese Abströmschlitze die Ansprechzeit des Gassensors 100 deutlich verkürzten können. So zeigt die Achse Y des Diagramms den gemessenen Strom in µA und die Achse X die Zeit in Sekunden. Im Diagramm sind drei Messkurven I, II und III dargestellt. Die Messkurve I zeigt das Ansprechverhalten eines Gassensors 100 mit einer Vorrichtung 10, welche Abströmschlitze 211 aufweist. Die Messkurven II und III zeigen das Ansprechverhalten eines Gassensors 100 mit einer Vorrichtung 10 ohne Abströmschlitze 211. Man erkennt das deutlich früher und höher einsetzende Signal der Messkurve I gegenüber den Messkurven II und III.

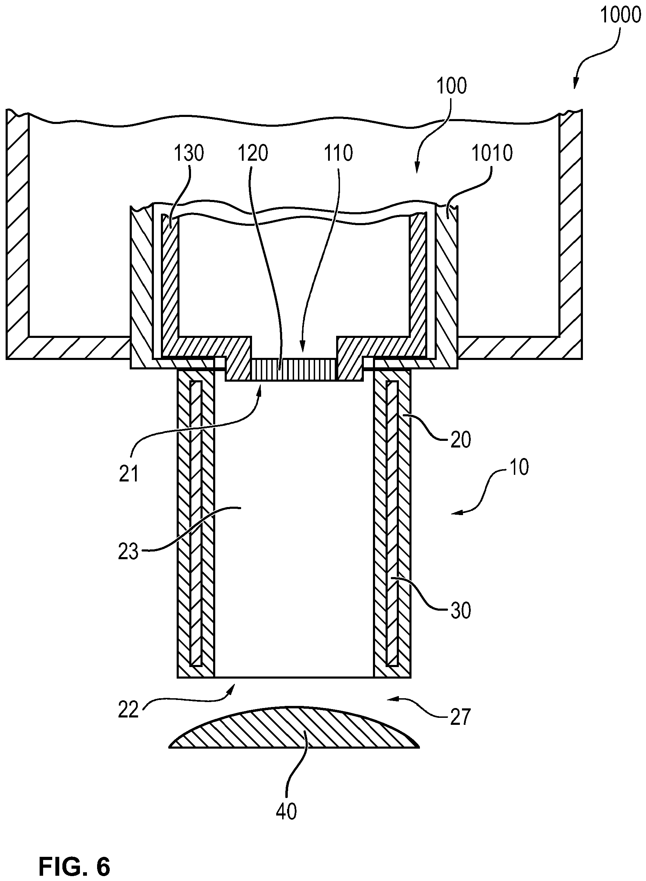

Die Figur 6 zeigt darüberhinausgehend eine Ausführungsvariante, bei welcher der Gassensor 100 in eine Aufnahme 1010 eines Gaswarn- oder Gaskonzentrationsmessgerätes 1000 eingesetzt ist. Man erkennt, dass die Vorrichtung 10 auch am Gehäuse 1020 des Gaswarn- oder Gaskonzentrationsmessgerätes 1000 montiert sein kann oder die Aufnahme 1010 umfassen kann. In jedem Fall gilt auch in Bezug auf die Figuren 5a, 5b und 6, dass die Vorrichtung 10 dem in den Figuren 1a, 1b und 1c gezeigten Ausführungsbeispiel entspricht. Es versteht sich jedoch von selbst, dass dies nur exemplarisch ist und dass anstelle dieser Variante der Vorrichtung 10 auch die in den Figuren 2a, 2b, 2c oder 3 dargestellte Variante am Gassensor 100 bzw. am Gaswarn- oder Gaskonzentrationsmessgerät 1000 montiert sein kann. Gleiche Bezugszeichen zeigen in allen Darstellungen gleiche oder einander entsprechende Bauteile.

Die Erfindung ist nicht auf eine der vorbeschriebenen Ausführungsformen beschränkt, sondern in vielfältiger Weise abwandelbar.

Das Heizelement 30 kann abweichend von Fig. 1c und Fig. 5b auch nicht entlang des gesamten Umfanges angeordnet sein, sondern nur punktuell oder asymmetrisch in der Wand des Gehäuses 20 eingelassen sein.

**Bezugszeichenliste**

| | | | |
|---|---|---|---|
| 10 | Vorrichtung | 30 | Heizelement |
| 20 | Gehäuse | 40 | Prallschutzteller |
| 21 | Austrittsöffnung | 41 | Oberseite |
| 211 | Abströmschlitz | 42 | Unterseite |
| 212 | Haltesteg | 43 | ringförmiges Element |
| 22 | Öffnung | 44 | ringförmiges Element |
| 23 | Strömungsberuhigungsraum | 100 | Gassensor |
| 231 | sensornahes Ende | 110 | Zuströmbereich |
| 232 | sensorfernes Ende | 120 | Schutzfiltervorrichtung |
| 24 | Haltesteg | 130 | Gehäuse |
| 25 | Außenwand | 1000 | Gaswarn- oder Gaskonzentrationsmessgerät |
| 26 | Innenwand | 1010 | Aufnahme |
| 27 | Zutrittsöffnung | 1020 | Gehäuse |

## Patentansprüche

1. Vorrichtung zur Feuchte- und Partikelabscheidung (10) für einen Gassensor (100), wobei die Vorrichtung (10) ein Gehäuse (20) aufweist, in welchem ein Strömungsberuhigungsraum (23) ausgebildet ist, wobei der Strömungsberuhigungsraum (23) ein sensornahes Ende (231) und ein sensorfernes Ende (232) aufweist, wobei am sensorfernen Ende (232) eine Zutrittsöffnung (27) ausgebildet ist und wobei am sensornahen Ende (231) eine Austrittsöffnung (21) ausgebildet ist, wobei die Vorrichtung wenigstens ein Heizelement (30) aufweist und der Strömungsberuhigungsraum (23) durch das wenigstens eine Heizelement (30) derart beheizbar ist, dass während des Betriebes der Vorrichtung (10) zwischen dem sensornahen Ende (231) und dem sensorfernen Ende (232) ein Temperaturgradient ausgebildet ist, und wobei die Vorrichtung einen Prallschutz aufweist, der einen Prallschutzteller (40) umfasst
**dadurch gekennzeichnet, dass**
in der Wand des Gehäuses (20) das wenigstens eine Heizelement (30) angeordnet ist.

2. Vorrichtung nach Anspruch 1, **dadurch gekennzeichnet, dass** die Innenwand (26) des Strömungsberuhigungsraumes (23) eine kondensationsverhindernde Oberfläche aufweist.

3. Vorrichtung nach einem der vorhergehenden Ansprüche, **dadurch gekennzeichnet, dass** die Heizleistung des wenigstens einen Heizelementes (30) ungleichmäßig über den Umfang des Gehäuses (20) verteilt ist.

4. Vorrichtung nach Anspruch 3, **dadurch gekennzeichnet, dass** der Prallschutz wenigstens ein ringförmiges Element (43) umfasst, das zwischen dem Prallschutzteller (40) und dem sensorfernen Ende (232) angeordnet ist.

5. Vorrichtung nach einem der Ansprüche 3 oder 4, **dadurch gekennzeichnet, dass** der Prallschutz mehrere ringförmige Elemente (43, 44) umfasst, die zwischen dem Prallschutzteller (40) und dem sensorfernen Ende (232) angeordnet sind.

6. Gassensor (100) mit einer Vorrichtung zur Feuchte- und Partikelabscheidung (10) entsprechend einem der vorhergehenden Ansprüche.

7. Gassensor nach Anspruch 6, **dadurch gekennzeichnet, dass** das Gehäuse (20) der Vorrichtung zur Feuchte- und Partikelabscheidung (10) stoffschlüssig mit dem Gehäuse (130) des Gassensors (100) verbunden ist.

8. Gassensor nach Anspruch 6, **dadurch gekennzeichnet, dass** das Gehäuse (20) der Vorrichtung zur Feuchte- und Partikelabscheidung (10) kraft- oder formschlüssig mit dem Gehäuse (130) des Gassensors (100) verbunden ist.

9. Gassensor nach einem der Ansprüche 6 bis 8, **dadurch gekennzeichnet, dass** zwischen dem Gehäuse des Gassensors und dem Gehäuse der Vorrichtung Abströmschlitze ausgebildet sind.

10. Gassensor nach einem der Ansprüche 6 bis 9, **dadurch gekennzeichnet, dass** das Volumen des Strömungsberuhigungsraumes derart bemessen ist, dass die Ausbildung eines Konzentrationsgradienten des Analyten zwischen der Zutrittsöffnung und dem sensornahen Ende verhindert wird.

11. Gaswarn- oder Gaskonzentrationsmessgerät (1000) mit einem elektrochemischen Gassensor (100) und einer Vorrichtung zur Feuchte- und Partikelabscheidung (10) entsprechend einem der vorhergehenden Ansprüche.

12. Gaswarn- oder Gaskonzentrationsmessgerät (1000) nach Anspruch 11, **dadurch gekennzeichnet, dass** der Gassensor in einer Aufnahme des Gaswarn- oder Gaskonzentrationsmessgerätes angeordnet ist und die Vorrichtung stoff-, kraft- oder formschlüssig mit der Aufnahme verbunden ist, so dass das sensornahe Ende der Vorrichtung dem Gaseinlass des Gassensors zugewandt ist und/oder diesen umfasst.

## Claims

1. Device for separation of moisture and particles (10) for a gas sensor (100), wherein the device (10) has a housing (20) in which a flow calming chamber (23) is formed, wherein the flow calming chamber (23) has a near-sensor end (231) and a sensor-remote end (232), wherein an inlet opening (27) is formed at the sensor-remote end (232) and wherein an outlet opening (21) is formed at the near-sensor end (231), wherein the device has at least one heating element (30) and the flow calming chamber (23) is heatable by the at least one heating element (30) in such a way that, during the operation of the device (10), a temperature gradient is formed between the near-sensor end (231) and the sensor-remote end (232), and wherein the device has an impact guard comprising an impact guard plate (40),
**characterized in that**
the at least one heating element (30) is disposed in the wall of the housing (20).

2. Device according to Claim 1, **characterized in that** the inner wall (26) of the flow calming chamber (23) has a condensation-preventing surface.

3. Device according to either of the preceding claims, **characterized in that** the heating output of the at least one heating element (30) is distributed inhomogeneously over the circumference of the housing (20).

4. Device according to Claim 3, **characterized in that** the impact guard comprises at least one annular element (43) disposed between the impact guard plate (40) and the sensor-remote end (232).

5. Device according to either of Claims 3 and 4, **characterized in that** the impact guard comprises multiple annular elements (43, 44) disposed between the impact guard plate (40) and the sensor-remote end (232).

6. Gas sensor (100) having a device for moisture and particle separation (10) according to any of the preceding claims.

7. Gas sensor according to Claim 6, **characterized in that** the housing (20) of the device for moisture and particle separation (10) is cohesively bonded to the housing (130) of the gas sensor (100).

8. Gas sensor according to Claim 6, **characterized in that** the housing (20) of the device for moisture and particle separation (10) is force-fittingly or form-fittingly bonded to the housing (130) of the gas sensor (100).

9. Gas sensor according to any of Claims 6 to 8, **characterized in that** outflow slits are formed between the housing of the gas sensor and the housing of the device.

10. Gas sensor according to any of Claims 6 to 9, **characterized in that** the volume of the flow calming chamber is such that the formation of a concentration gradient of the analyte is prevented between the inlet opening and the near-sensor end.

11. Gas detector or gas concentration meter (1000) having an electrochemical gas sensor (100) and a device for moisture and particle separation (10) according to any of the preceding claims.

12. Gas detector or gas concentration meter (1000) according to Claim 11, **characterized in that** the gas sensor is disposed in a receptacle in the gas detector or gas concentration meter and the device is connected to the receptacle in a cohesive, force-fitting or form-fitting bond, such that the near-sensor end of the device faces and/or encompasses the gas inlet of the gas sensor.

## Revendications

1. Dispositif (10) destiné à un capteur de gaz (100) et affecté à la séparation d'humidité et de particules, ledit dispositif (10) étant muni d'un boîtier (20) dans lequel est formée une chambre (23) de stabilisation d'écoulement, laquelle chambre (23) de stabilisation de l'écoulement comporte une extrémité (231) proche du capteur et une extrémité (232) éloignée dudit capteur, une ouverture d'afflux (27) et une ouverture de sortie (21) étant ménagées, respectivement, au niveau de ladite extrémité (232) éloignée du capteur et au niveau de ladite extrémité (231) proche dudit capteur, sachant que le dispositif est pourvu d'au moins un élément chauffant (30) et que la chambre (23) de stabilisation de l'écoulement peut être chauffée, par ledit élément chauffant (30) à présence minimale, de façon telle qu'un gradient de température soit formé entre l'extrémité (231) proche du capteur et l'extrémité (232) éloignée dudit capteur, au cours du fonctionnement dudit dispositif (10), et sachant que ledit dispositif est doté d'une protection antichocs incluant une plaque (40) de protection antichocs,
**caractérisé par le fait que**
l'élément chauffant (30), à présence minimale, est implanté dans la paroi du boîtier (20).

2. Dispositif selon la revendication 1, **caractérisé par le fait que** la paroi intérieure (26) de la chambre (23) de stabilisation de l'écoulement présente une surface prévenant la condensation.

3. Dispositif selon l'une des revendications précédentes, **caractérisé par le fait que** la puissance de chauffage de l'élément chauffant (30), à présence minimale, est irrégulièrement répartie sur le pourtour du boîtier (20).

4. Dispositif selon la revendication 3, **caractérisé par le fait que** la protection antichocs inclut au moins un élément annulaire (43) interposé entre la plaque (40) de protection antichocs et l'extrémité (232) éloignée du capteur.

5. Dispositif selon l'une des revendications 3 ou 4, **caractérisé par le fait que** la protection antichocs comprend plusieurs éléments annulaires (43, 44), interposés entre la plaque (40) de protection antichocs et l'extrémité (232) éloignée du capteur.

6. Capteur de gaz (100) équipé d'un dispositif (10) correspondant à l'une des revendications précédentes, affecté à la séparation d'humidité et de particules.

7. Capteur de gaz selon la revendication 6, **caractérisé par le fait que** le boîtier (20) du dispositif (10) affecté à la séparation d'humidité et de particules est relié matériellement au carter (130) dudit capteur de gaz (100).

8. Capteur de gaz selon la revendication 6, **caractérisé par le fait que** le boîtier (20) du dispositif (10) affecté à la séparation d'humidité et de particules est relié au carter (130) dudit capteur de gaz (100) par engagement positif, ou par complémentarité de formes.

9. Capteur de gaz selon l'une des revendications 6 à 8, **caractérisé par le fait que** des fentes d'évacuation sont pratiquées entre le carter dudit capteur de gaz et le boîtier du dispositif.

10. Capteur de gaz selon l'une des revendications 6 à 9, **caractérisé par le fait que** le volume de la chambre de stabilisation de l'écoulement est dimensionné de manière à empêcher la formation d'un gradient de concentration de l'analyte entre l'ouverture d'afflux et l'extrémité proche dudit capteur.

11. Appareil (1000) d'alerte au gaz ou de mesure de concentrations gazeuses, équipé d'un capteur de gaz (100) électrochimique et d'un dispositif (10) correspondant à l'une des revendications précédentes, affecté à la séparation d'humidité et de particules.

12. Appareil (1000) d'alerte au gaz ou de mesure de concentrations gazeuses, selon la revendication 11, **caractérisé par le fait que** le capteur de gaz est implanté dans un logement dudit appareil d'alerte au gaz ou de mesure de concentrations gazeuses, et le dispositif est relié audit logement par solidarisation matérielle, par engagement positif ou par complémentarité de formes, de telle sorte que l'extrémité dudit dispositif, proche dudit capteur, soit tournée vers l'admission de gaz dans ledit capteur de gaz et/ou inclue cette dernière.
